# EUROPEAN PATENT APPLICATION

(11) **EP 0 938 879 A2**
(43) Date of publication of application: **01.09.1999**
(21) Application number: 99301340.8
(22) Date of filing: 24.02.1999
(51) Int. Cl.: A61F 2/06

(54) **Stent-graft assembly and method of manufacture**

(30) Priority: 25.02.1998 US 38479; 31.03.1998 US 53145
(71) Applicant: Medtronic Ave, Inc., Santa Rosa, California 95403 (US)
(72) Inventor: Williams, Michael S., Santa Rosa, CA 95409 (US); Nolting, John E., Santa Rosa, CA 95403 (US); Birdsall, Matthew J., Santa Rosa, CA 95404 (US); Lashinski, Robert D., Sebastopol, CA 95472 (US); Shull, Samuel L., Santa Rosa, CA 95401 (US)
(74) Representative: Bayliss, Geoffrey Cyril

(57) **Abstract**

A stent-graft assembly and method of preparing the same are disclosed. In a first embodiment, the assembly comprises a stent, a coating and a membrane, wherein the coating and membrane are bonded to one another at a plurality of bonding regions such that they define a unitary structure, thereby permanently adhering the coating and membrane to one another. A second embodiment of the stent-graft assembly comprises a stent, a coating and a porous membrane, wherein portions of the coating extend into the pores of the porous membrane to sealingly engage the membrane, again to achieve secure adhesion. In either embodiment, the stent-graft assembly may comprise an elastomeric coating substantially encapsulating the entire stent, and a membrane covering only the medial portion of the assembly. The diameter of the proximal and distal regions may thereby be variable over a wider range than the medial portion, enhancing conformity of the effective diameter of the assembly with the diameter of the body lumen and ensuring that the device will securely seat and seal within the body lumen. The proximal and distal regions of the stent-graft assembly may comprise an additional coating, whereby layers of material are sealed, thereby minimizing thrombogenic potential of free ends of the assembly. In any of the foregoing embodiments, the membrane may be a thin-walled tube, and the method of making such a tube is also disclosed.

## Description

The present invention relates generally to endoluminal grafts for stenotic or diseased lumens and methods of making such grafts. More particularly, the invention includes a stent-graft assembly and methods of making a stent-graft assembly by coating the stent members and bonding the graft layer to the coating.

A wide range of medical treatments have been previously developed using "endoluminal prostheses," which terms are herein intended to mean medical devices which are adapted for temporary or permanent implantation within a body lumen, including both naturally occurring or artificially made lumens. Examples of lumens in which endoluminal prostheses may be implanted include, without limitation: arteries, such as those located within the coronary, mesentery, peripheral, or cerebral vasculature; veins; gastrointestinal tract; biliary tract; urethra; trachea; hepatic shunts; and fallopian tubes. Various types of endoluminal prostheses have also been developed, each providing a uniquely beneficial structure to modify the mechanics of the targeted luminal wall.

For example, various grafts, stents, and combination stent-graft prostheses have been previously disclosed for implantation within body lumens. More specifically regarding stents, various designs of these prostheses have been previously disclosed for providing artificial radial support to the wall tissue which forms the various lumens within the body, and often more specifically within the blood vessels of the body. An example of such a stent displaying optimal radial strength includes, but is not limited to, the stent disclosed in U.S. Patent No. 5,292,331 to Boneau, the disclosure of which is herein incorporated by reference. Stents of other designs are known in the art, and may also be suitable for use in the stent-graft assembly. Other example of stents include but are not limited to those disclosed in U.S. Patent No. 4,733,665 issued to Palmaz, U.S. Patent No. 5,195,984 issued to Schatz, or U.S. Patent No. 5,514,154 issued to Lau. Stents are used alone or in conjunction with grafts.

The use of an angioplasty balloon catheter is common in the art for treatment to enlarge a stenotic or diseased blood vessel. This treatment is known as percutaneous transluminal angioplasty, or PTA. To provide radial support to the treated vessel in order to prolong the positive effects of PTCA, a stent may be implanted, with the use of a PTA balloon catheter, within the affected lumen.

Under this procedure, the stent may be collapsed to an insertion diameter and inserted into a body lumen at a site remote from the diseased vessel. The stent may then be delivered to the desired site of treatment within the affected lumen and deployed to its desired diameter for treatment. Although the stents listed above are balloon expandable, stents which rely on other modes of deployment such as self-expansion, may be used to make a device according to the present invention. Because the procedure requires insertion of the stent at a site remote from the site of treatment, the device must be guided through the potentially tortuous conduit of the body lumen to the treatment site. Therefore, the stent must be capable of being reduced to a small insertion diameter and must be flexible.

However, during an angioplasty procedure, atheromatous plaques undergo fissuring, thereby creating a thrombogenic environment in the lumen. Excessive scarring may also occur following the procedure, potentially resulting in reocclusion of the treated lumen. Attempts to address these problems include providing a suitable surface within the lumen for more controlled healing to occur in addition to the support provided by a stent. These attempts include providing a lining or covering in conjunction with an implanted stent. A stent with such a lining or covering is known in the art as a stent-graft.

The membrane of a stent-graft may prevent excessive tissue prolapse or protrusion of tissue growth through the interstices of the stent while allowing limited tissue in-growth to occur to enhance the implantation. The surface of the graft material at the same time minimizes thrombosis, prevents scarring from occluding the lumen and minimizes the contact between the fissured plaque and the hematological elements in the bloodstream.

An additional challenge in implanting stents and stent-grafts includes secure placement of the device within the body lumen. The device must be securely seated within the vessel to prevent migration of the device following implantation, which could prove catastrophic.

A stent-graft must also be adequately sealed to ensure blood flow through the device and to minimize leakage of blood between the stent-graft and the diseased portion of the lumen, otherwise known as perigraft leakage. Consequently, adequate seating and sealing of a stent-graft within the lumen is necessary.

Sufficient seating and sealing of prior art devices allows little margin of error in the measurement of the interior of the lumen to be treated. The commonly irregular interior of the diseased lumen may decrease the accuracy of the measurement of the lumen to be treated. It is therefore an additional objective of such inventions to provide a device which will securely seat and seal within the lumen regardless of the irregularities of the interior surface of the lumen and with some margin of error allowed for the measurement of the lumen.

Other objectives of a combination stent-graft may include delivering therapeutic agents via the assembly, excluding aneurysms or other malformations, occluding a side branch of a lumen without sacrificing perforator branches, conferring radiopacity on the device, and others. Various designs to achieve these objectives include stents partially or completely coated or covered with materials, some of which are impregnated with therapeutic agents, radiopaque elements, or other features designed to achieve the particular objectives of the device.

A graft component may be combined with a stent in order to achieve some or all of the foregoing objectives. However, adding a graft layer to the stent increases the challenges of delivering a stent via a catheter by increasing the crossing profile, or diameter, of the device, and by decreasing the flexibility of the device. Because the angioplasty process requires the insertion of the device into a body lumen at a site remote from the site of treatment and the guiding of the device the body lumen to the treatment site, it is required that the device be both capable of being collapsed to a relatively small diameter and be quite flexible. Moreover, flexibility and a desirable insertion diameter must be achieved without sacrificing the treatment objectives of the assembly, which include, at a minimum, radial strength. Therefore, an objective of a combination stent and graft is achieving the advantages of both a stent and a graft without significantly increasing the crossing profile of the device or significantly decreasing the flexibility of the device.

Various methods of manufacturing graft devices alone have been disclosed in the art. One such method for manufacturing a graft is disclosed in U.S. Patent No. 5,641,373, issued to Shannon et al. The disclosed method comprises reinforcing an extruded flouropolymer tube with a second flouropolymer tube. The second tube is prepared by winding fluoropolymer tape around the exterior of a mandrel and heating it to form a tube. The graft may then be mounted on an anchoring mechanism such as a stent or other fixation device. Various designs to achieve these objectives include stents coated or covered with materials which are impregnated with therapeutic agents.

A specific example of a coated stent is disclosed by Pinchuk in European Patent Application EP 0 797 963 A2. The objectives of Pinchuk's invention include both increasing the hoop strength and decreasing the thrombogenic potential of a criss-crossed wire stent or a zig-zag stent. Pinchuk's application discloses coating the stent with a polymer to prevent the scissoring action of the wires without occluding the interstices of the stent lattice with the coating.

An example of a stent and tubular graft is disclosed in U.S. Patent No. 5,522,882 issued to Gaterud, et al. Gaterud discloses an expandable stent mounted on a balloon and a graft mounted over the stent.

U.S. Patent No. 5,123,917 issued to Lee claims a flexible and expandable inner tube upon which discusses separate ring scaffold members are mounted, and a flexible and expandable outer tube enclosing the inner tube and scaffold members. The rings may be secured to the inner liner with an adhesive layer. Alternatively, the liners may be adhered to each other with the rings trapped between the layers. Lee discloses that the luminal surface of the device may be coated with various pharmacological agents.

Similarly, U.S. Patents No. 5,282,823 and 5,443,496, both issued to Schwartz, et al. disclose a stent with a polymeric film extending between the stent elements, and strain relief means in the form of cuts in the film to allow the stent to fully expand and conform to the interior of the lumen. The thin polymeric film is applied to the stent while in solution and dried. Once dried, cuts are made in the film to provide strain relief means.

Another assembly includes a stent embedded in a plastic sleeve or stitched or glued to a nylon sleeve, as in U.S. Patent No. 5,507,771, issued to Gianturco. Other prior art devices requiring stitching of the graft to the stent are disclosed in European Patent Application EP 0 686 379 A2, which teaches a perforate tubular frame having a fabric liner stitched to the frame, and World Intellectual Property Organization Application Number WO 96/21404 which indicates that the graft be stitched to the stent, and possibly to loops or eyelets which are part of the stent structure.

Numerous devices in the prior art attempt coupling the stent and graft material through either adhesives or heat bonding. U.S. Patent No. 5,637,113 issued to Tartaglia, and U.S. Patent No. 5,653,747 issued to Dereume both teach a stent with a sheet of polymeric film wrapped around the exterior. Tartaglia teaches that the film is attached to the stent at one end by an adhesive, by a hook and notch arrangement, or by dry heat sealing. The polymer can also be attached to the stent by wrapping the film circumferentially around the stent and attaching the polymer film to itself to form a sleeve around the stent by heating and melting the film to itself, adhesive bonding, solvent bonding, or by mechanical fastening, such as by a clip. The film may be loaded or coated with a therapeutic agent.

U.S. Patent No. 5,628,788, issued to Pinchuk, discloses a process of melt-attaching a graft to a stent by disposing a layer of material between the stent and graft which has a lower melting point than the graft, and heating the assembly to the melting point of the low-melting point material. Pinchuk also teaches adhering a textile graft to a stent by coating a stent with vulcanizing silicone rubber adhesive and curing the adhesive. Pinchuk discloses a similar stent-graft assembly in European Patent Application EP 0 689 805 A2 and teaches that the graft member can be bonded to the stent member thermally or by the use of adhesive agents.

Similarly, in World Intellectual Property Organization Application No. WO 95/05132 discloses a stent with and inner and/or and outer liner wrapped around the stent to form a seam, with the liner(s) affixed using an adhesive or melt-attached using a layer of a material with a lower melting point.

U.S. Patent 5,645,559, issued to Hachtman, et al., discloses a multiple layer stent-graft assembly comprising a first layer defining a hollow tubular construction, a second layer having a self-expanding braided mesh construction and a layer of polymeric material disposed between the first and second layers. The polymeric material may be adhered by two-sided adhesive tape. The self-expanding braided mesh, the tubular material, or both may be larger in diameter in the distal regions than in the medial region.

U.S. Patent No. 5,534,287, issued to Lukic, discloses methods which result in a covered stent, the covering adhered via a lifting medium.

U.S. Patent No. 5,674,241, issued to Bley, et al. teaches that a hydrophilic polymer layer may be laminated, imbedded, coated, extruded, incorporated, or molded around an expandable mesh stent while the stent is in its collapsed condition, and the stent and graft permitted to expand upon hydration.

U.S. Patent No. 5,628,786, issued to Banas, discloses a polytetrafluoroethylene graft which has a reinforcing structure integrally bound to the graft. The reinforcing structure may be in the form of a rib which is sintered or otherwise integrally bound to the graft.

U.S. Patent No. 5,207,960, issued to Moret de Rocheprise, discloses a process for the manufacture of a thin-walled tube of fluorinated resin tape. The method includes winding the tape around a mandrel and sintering the tape. While still on the mandrel, the tube is rolled to elongate the tube, to reduce the thickness of the tube, and to facilitate removal of the tube from the mandrel. The patent discloses that the tubes obtained can be used particularly as sheaths for the lining of metal tubes.

There are also numerous examples of combination stent-grafts disclosed in the art. U.S. Patent No. 5,653,747 issued to Dereume discloses a stent to which a graft is attached. The graft component is produced by extruding polymer in solution into fibers from a spinnerette onto a rotating mandrel. A stent may be placed over the fibers while on the mandrel and then an additional layer of fibers spun onto the stent. The layer or layers of fibers may be bonded to the stent and/or one another by heat or by adhesives.

Challenges arising in the art which none of the prior art adequately addresses include minimizing, if not eliminating, migration of the stent, graft or stent-graft; minimizing, if not eliminating, delamination of the stent and graft material; minimizing thrombogenic potential, vessel reocclusion and tissue prolapse following deployment; and achieving a stent-graft assembly of sufficiently small crossing profile and which is sufficiently flexible. Shortcomings associated with the prior art include: inadequate adhering of coatings and coverings to stents; inadequate adhering of coatings to coverings; inability to attain sufficient seating and sealing of the device within the vessel; failure to shield the injured vascular surface; failure to prevent tissue ingrowth from occluding the lumen; failure to minimize the embolization of particles loosely adherent to the vessel wall, (especially during device placement and deployment); increased thrombogenic potential arising from delamination of the stent and graft material; and failing to provide safeguards for the accurately estimating and achieving the required range of expansion of the diameter of the device *in vivo.*

The present invention and its varied embodiments address several problems associated with the prior art. It is a first objective of this invention to provide an improved stent-graft assembly for the repair and support of a body lumen. It is a second objective of this invention to provide an improved stent-graft assembly with ample radial strength and minimal thrombogenic potential without appreciably increasing the profile of the device over that of the stent alone.

It is a further objective of this invention to solve the problem in the prior art of inadequate adhesion between the stent and graft material. It is also an objective of this invention to provide an improved stent-graft assembly by utilizing optimal combinations of and configurations of materials and methods to achieve optimal bonding between the stent and graft materials.

An additional objective of this invention is to balance the need for some tissue in-growth against the need to minimize thrombogenic potential and excessive cell growth through the interstices of the stent. This objective is achieved by providing a non-thrombogenic stent-graft assembly which is a smoother device, especially in regions where the prior art stent-graft has a tendency to fray, delaminate, or exhibit a scissoring effect. This objective is also achieved by providing a stent-graft assembly which shields the injured vascular surface, blocks excessive tissue in-growth through the stent, and minimizes the embolization of particles loosely adherent to the vessel wall especially during placement and deployment of the device. The device can also be used to control the luminal protrusion of dissection planes created during PTA or spontaneous fissuring.

It is a further object of this invention to provide a stent-graft assembly which is capable of delivering therapeutic modalities including but not limited to pharmaceuticals, gene therapy, and radiation therapy.

And finally, it is an object of this invention to address the need to provide a stent-graft assembly with some flexibility in the range of effective diameter of the device *in vivo.*

A stent-graft assembly according to the present invention first comprises a generally cylindrical stent which comprises a plurality of stent segments each having a plurality of support members. Some or all of the support members comprise a coating which substantially encapsulates the coated support members. Further to the coating, the stent-graft comprises a membrane or covering which may be ultra-thin, which is bonded to the coating. The material used for the membrane may comprise an ultra-thin polymer. In one embodiment, the proximal and distal regions of the stent-graft have an additional coating over the first coating and the membrane. In an alternative embodiment, the proximal and distal regions of the stent may be left completely uncoated and uncovered if needed for the particular medical application of the device.

The membrane may be either on the inner or the outer surface of the stent or both. The material used for the membrane comprises, according to the particular embodiment, a material which is chemically similar to that of the coating, and/or a porous material. In the embodiment in which the coating and membrane are of similar materials, the means for adhering the membrane to the coating of the stent comprises a plurality of bonding regions, generally where the membrane comes into contact with the coating on the support members, in which the materials unite to form a unitary structure. In the embodiment comprising a porous membrane, the membrane is adhered as a result of extensions of the coating into the pores of the membrane and the resulting interlocking engagement between the coating extensions and the pores of the membrane.

In an alternate embodiment of the invention, the entire stent-graft is coated with an elastomeric material, and the medial region of the stent-graft comprises either an elastomeric or a non-elastomeric membrane bonded with the coating. Finally, the proximal and distal regions of the assembly have a second membrane which is elastomeric. Suitable materials for the second membrane include any which are sufficiently elastic, such as urethanes, latexes, polysiloxanes, and others. In this embodiment, the membrane is securely bound to the coating as described in reference to the foregoing embodiments. *In vivo,* during deployment, the proximal and distal regions of the stent-graft are expanded to conform to the diameter of the interior of the vessel, thereby effectively seating and sealing the stent-graft within the vessel.

Another embodiment of the invention comprises a stent with a membrane on the interior of the stent and a membrane on the exterior of the stent, the membranes bound to one another through the interstices of the stent. The membranes may be sintered or otherwise bound to one another. The proximal and distal regions of the stent comprise a coating which substantially encapsulates the assembly at the proximal and distal regions.

The method according to a first embodiment of the present invention comprises helically wrapping ultra-thin polymeric tape around a mandrel, adjusting the angle of orientation of the tape to the mandrel depending upon the desired distensibility of the membrane and allowing adjacent edges of the helical wrapping to overlap somewhat; sintering the tape to itself over the mandrel to produce a thin tube; removing the thin tube from the mandrel; coating a stent with a polymer; covering and/or lining the coated stent with the thin tube; introducing a solvent to attach the coating to the membrane; curing the assembly to drive off remaining solvent.

The method according to an alternative embodiment comprises winding the polymeric tape around the mandrel to form two layers, in each layer reversing the angle of orientation of the tape to the longitudinal axis of the mandrel to form a bias ply, and then following the remaining steps of the method described above. In any given embodiment, the angle of orientation of the tape to the longitudinal axis of the mandrel, dependent on the width of tape and diameter of the mandrel, can be varied depending upon the desired distensibility of the graft component of the device. The sintering parameters can also be varied to affect the distensibility of the device. Further, pressure may be utilized in conjunction with sintering to improve the adherence of the tape. And finally, the amount of overlap between adjacent edges of tape can be varied depending upon the particular indication for the device.

In yet a further embodiment of a method according to the invention, a thin-walled stent-graft assembly, having inner and outer membranes, can be fabricated utilizing pressure and heat.

The method according to a first embodiment of the present invention comprises coating a stent; covering or lining the coated stent with a membrane possessing chemical properties which are the same, substantially similar or similar to the chemical properties of the coating; introducing a solvent such that the coating and membrane partially dissolve and, where they contact one another, enter solution together and essentially unite to define a continuous structure; and then removing the solvent. The method according to an alternative embodiment comprises coating a stent; covering or lining the coated stent with a porous membrane; introducing an appropriate solvent which acts on the coating such that the coating becomes of sufficient viscosity to migrate into the pores of the membrane; and removing the solvent.

In any of the methods according to the particular embodiment, the extent that the coating and membrane cover the stent can be varied. Also according to the particular embodiment, the proximal and distal regions of the stent-graft assembly may be coated a second time to seal the resulting layers of stent, coating and membrane and to substantially increase bond strength due to the increased surface area of the thicker encapsulation of the stent strut.

In the accompanying drawings:

Figure 1 illustrates a perspective view of a suitable stent for use in the present invention.

Figure 2 illustrates a perspective view with a progressive partial cut-away of an embodiment of the stent-graft assembly according to the present invention.

Figures 3A-3C illustrate a portion of the method of preparation of the membrane component of an embodiment of the stent-graft assembly according to the present invention.

Figures 4A and 4B show a cross-sectional view taken along line A-A of Figure 2.

Figure 5A-5D respectively illustrate the progressive results of the steps of one particular embodiment of the invention.

Figures 6A and 6B shows a cross-sectional view of a support member of an alternate embodiment of the present invention.

Figures 7A-7D respectively illustrate the progressive results of the steps of another particular embodiment of the invention.

Figure 8 illustrates an elevational view of another embodiment of a stent-graft assembly according to the present invention *in vivo* following deployment within a body lumen.

Figure 9 shows a perspective view of an embodiment of the invention which is used to provide radial support to a body lumen and to occlude a side branch or aneurysm of the same body lumen without sacrificing perforator branches.

Figure 10 illustrates a perspective view of a partial progressive cut-away of an alternative embodiment of the invention.

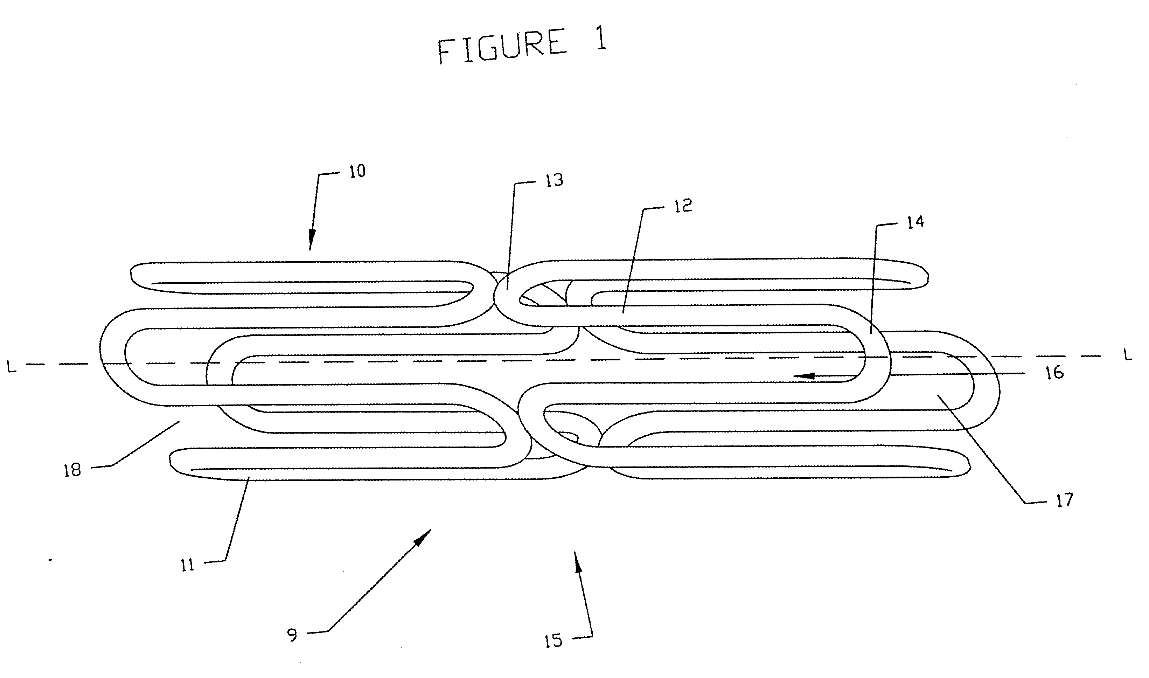

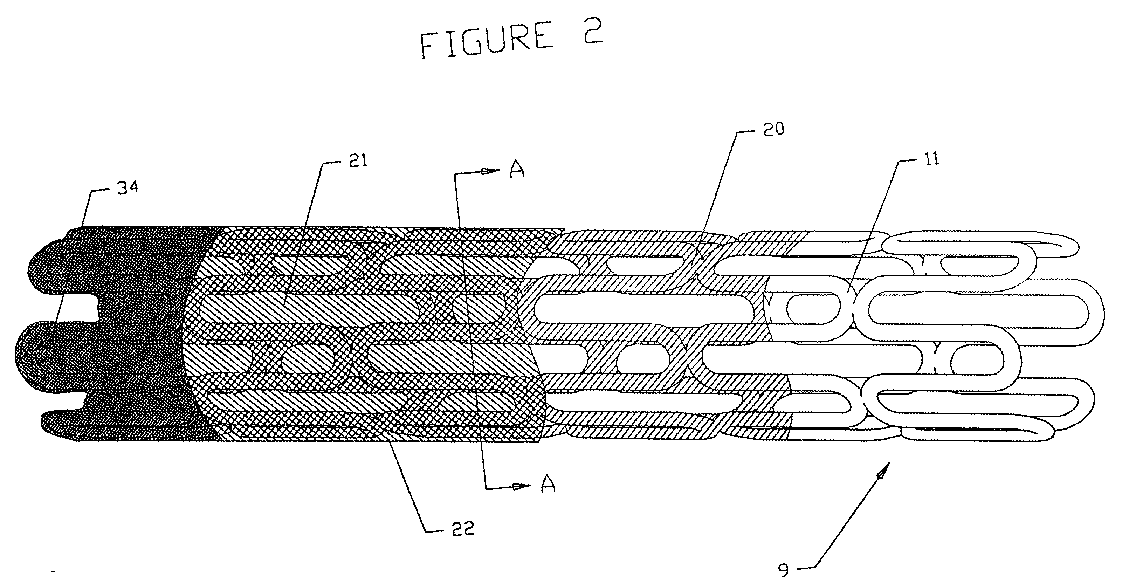

The stent-graft assembly according to the present invention is shown in Figure 2. One recommended stent for use as the stent member according to the present invention is shown at stent (9) in Figure 1. Other stents, such as those disclosed in U.S. Patent No.4,733,665 issued to Palmaz, U.S. Patent No. 5,195,984 issued to Schatz, or U.S. Patent No. 5,514,154 issued to Lau may also be suitable for use in the stent graft assembly.

Stent (9) is shown in Figure 1 to include a series of integrated, individual sinusoidal-shaped stent elements (10) which are each similar in design and construction to the stent assembly disclosed in U.S. Patent No. 5,292,331 to Boneau, the disclosure of which is herein incorporated by reference thereto. For the purpose of further illustration, however, the series of adjacent rings or circumferential wires form support members, such as is shown for the purpose of illustration at support member (11). Each ring is formed to include a serpentine shape which includes a plurality of peaks and struts extending longitudinally between adjacent peaks, such as is shown for example at strut (12) which extends between peaks (13,14). Each ring is further connected to an adjacent ring in at least one location where the peaks of their serpentine shape meet, resulting in an interconnected series of stent elements which forms generally cylindrical body (15). Cylindrical body (15) further forms a prosthesis passageway (16) extending through the plurality of adjacent, serpentine-shaped rings along longitudinal axis L and between proximal prosthesis port (17) and distal prosthesis port (18).

Further to the interconnected series of stent elements and their respective wire-like support members which form cylindrical body (15) as shown in Figure 1, spaces remain along cylindrical body (15) between adjacent peaks of each shaped ring and also between adjacent rings, particularly where the individual peaks of adjacent rings extend away from each other relative to longitudinal axis *L*.

Turning now to Figure 2, a stent-graft assembly according to one embodiment of the present invention comprises a stent (9) having a coating (20) on some or all of the rings or support members (11). The stent-graft assembly further comprises a membrane (21) which in this embodiment defines a vascular surface (22), but which in an alternate embodiment forms a luminal surface. The membrane may be less than 0.040 inch in thickness, and preferably is 0.00016 inch thick or less, and is distensible over a range of between 7 and 100 per cent over its primary unstretched diameter. The proximal and distal regions may comprise a second coating (34), as illustrated on the distal region only in Figure 2. Suitable material for the coating includes but is not limited to polyurethane, fluorinated ethylene propylene, and silicone. Suitable material for the membrane may be synthetic and includes but is not limited to polyesters, polytetrafluoroethylene, polyurethane and silicone. A therapeutic agent may be incorporated into the coating or the membrane using a number of different techniques known in the art, including loading, coating or laminating.

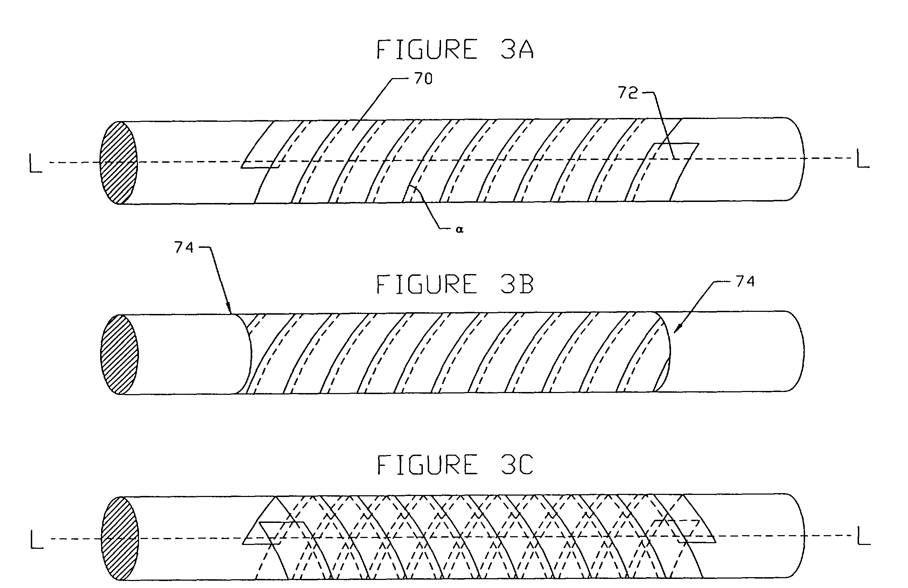

A portion of the method of preparation of the thin-walled graft component is illustrated in Figures 3A and 3B. Successive helical windings (70) of the thin polymeric tape (0.010 inches in thickness or less) (72) overlap to a desired extent. The amount of overlap can be varied depending upon the width of the tape, diameter of mandrel and the angle of the tape to the longitudinal axis of the mandrel. The invention contemplates that the adjacent edges of the tape overlap between 5 and 90 per cent of the width of the tape, with 10 to 60 per cent preferred. The finished graft component is distensible over a varying range depending upon the angle α of the tape to the longitudinal axis of the mandrel *L*. The lesser the angle α, the greater the radial distensibility. The degree of distensibility is also affected by the sintering process, and the parameters followed to achieve sintering of the tape may be varied depending upon the desired radial distensibility. The tape can be wound at any desired angle α to the longitudinal axis of the mandrel, more specifically, α≤90°. But in this embodiment the angle is preferably between 30 and 60 degrees. The tape is wound beginning from one end of the mandrel progressively to the other end of the mandrel. It can then be wound a second time in the reverse direction if an additional layer of polymeric tape is desired, such as shown in Figure 3C.

The wrapped mandrel is then subject to a sufficient temperature for a sufficient time to sinter the overlap layers together. For example, a PTFE wrapped mandrel is subject to a temperature of approximately 370 degrees C for between 30 and 45 minutes to sinter the overlapping portions together. Pressure may be utilized in conjunction with sintering to improve the adherence of the tape windings to one another.

An alternative sintering method for forming the thin-walled tube utilizes a radial compression process such as, hot isostatic compression. A preferred embodiment of which comprises placing the wrapped mandrel into a vial which is packed in a media such as, silica or other microbeads. A plunger is placed and held within the vial under pressure. The vial containing the wrapped mandrel under pressure is then heated at sufficient time and temperature to achieve sintering. As illustrated in Figure 3B, after sintering, the ends (74) of the newly formed tube are trimmed. Moreover, with respect to the use of non-PTFE tape, rather than sintering, the tape could be solvent bonded, UV bonded or the overlapping portions could be bonded together through the use of a pressure-sensitive adhesive.

The thin tube is then removed from the mandrel. If, because the tube constricts to some degree during sintering, difficulty is encountered in removing the tube from the mandrel, several methods to facilitate removing the tube may be used. Compressed air may be discharged at one end of the tube between the tube and the mandrel, or a flat tool may be used to loosen any temporary adhesion between the mandrel and the tube. Alternatively, a collapsible mandrel or a bar of reducible diameter may be used. Also, a lubricant such as silicone, can be introduced to facilitate removal of the tube from the mandrel.

The entire stent-graft assembly can also be fabricated in accordance with method for producing the thin-walled tube utilizing pressure and heat as discussed above. Specifically, a first tape is wrapped about the mandrel. Next, the stent is loaded onto the mandrel over the first tape. A second tape is then wrapped over the stent. The entire assembly is then placed into a vial which is packed with microbeads. The assembly is then subjected to pressure and heat for a sufficient time and at a sufficient temperature to achieve sintering. Additionally, rather than placing the assembly in a microbead-filled vial, the membranes of the assembly may be sintered together by placing the assembly in an oven at approximately 370°C.

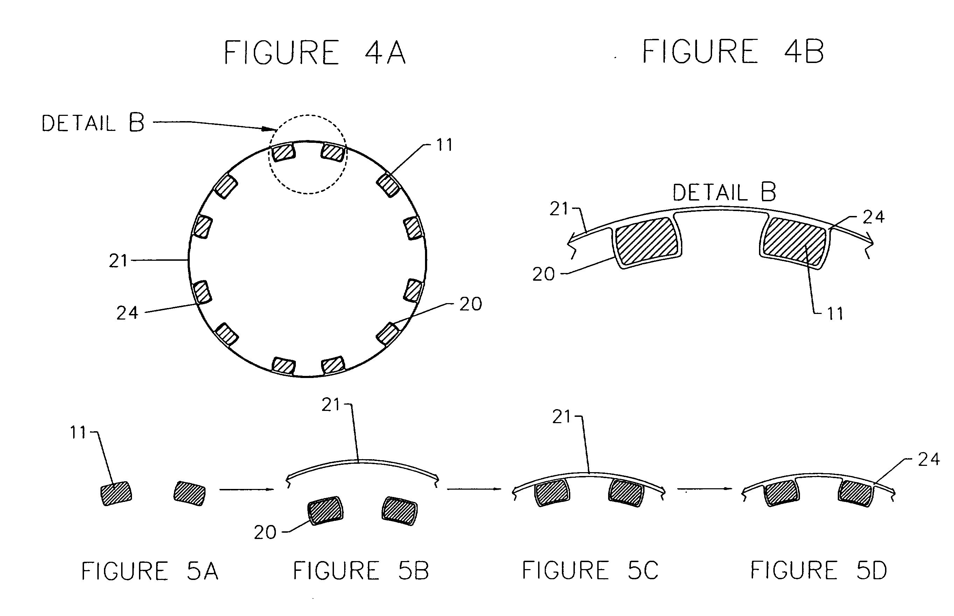

In the embodiment of the invention shown in Figures 4A and 4B, the materials comprising both the coating (20) and the membrane (21) are typically of chemically similar materials, such that when the assembly is subjected to a solvent, the coating and membrane partially dissolve. At the plurality of points at which they are in contact, the coating and membrane dissolve together to form bonding regions in which the coating and membrane unite to define a unitary structure (24). Although a non-porous membrane is depicted in Figures 4 through 5D, the membrane may be porous. Suitable solvents include any solvent which will degrade, dissolve or decrease the viscosity of the coating. Particularly suitable solvents include but are not limited to dimethyl acetamide, xylene, and isopropanol.

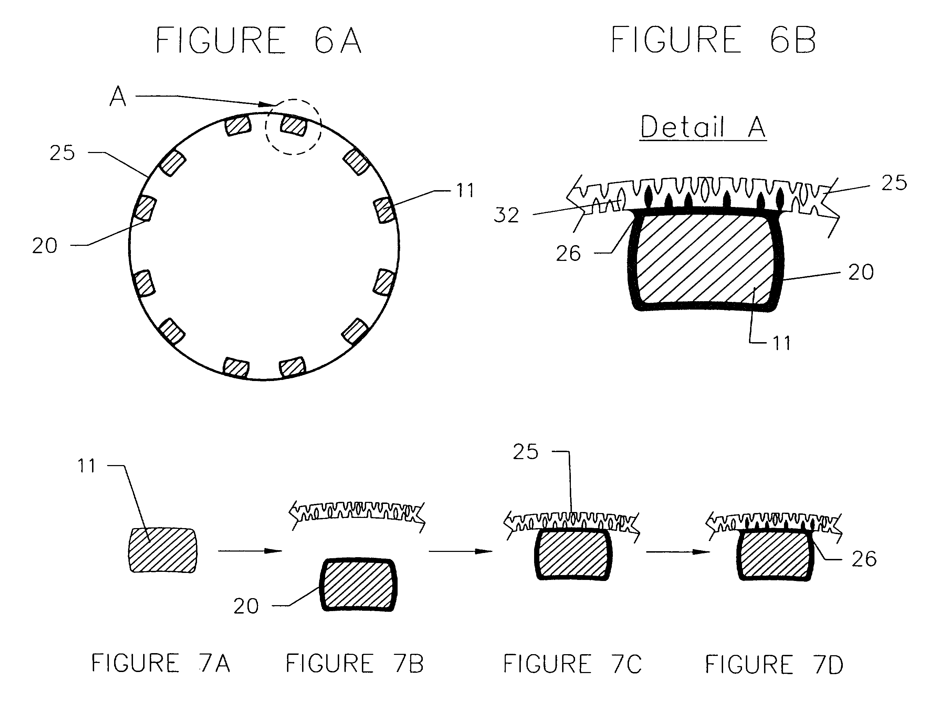

In another embodiment of the invention shown in Figure 6, the coating (20) and membrane (25) typically are not chemically similar materials. In this embodiment, the membrane comprises a plurality of pores (32). When the assembly is subjected to an appropriate solvent, the coating becomes decreasingly viscous. Subject to the ratio of the solvent to the coating and the relative porosity of the membrane, the coating in this form infiltrates the pores of the membrane to a varying extent. A plurality of bonding regions (26) at and beyond the surface of the membrane are formed where the coating fills the pores of the membrane and, after the assembly is cured to drive off the solvent, sealingly engages the membrane to the coating.

The sequential results of a first particular method of forming the stent-graft according to the present invention is shown illustratively following respective steps in Figures 5A-5D.

According to a first method, the stent-graft is prepared by first coating a stent via either a dipping process or a spraying process. In one embodiment, the spraying process is performed utilizing a 3.5% solids solution polyurethane in dimethyl acetamide. The newly coated stent is then cured to remove solvent. A cross-section of the resulting structure is illustrated in Figure 5B.

Following curing, the coated stent is loaded onto an expansion mandrel, is expanded to an intermediate diameter, and a polyurethane tube (21) is mounted over the exterior of the coated stent as shown in Figures 5B and 5C. The tube could alternatively be placed within the stent, or an additional tube may be placed within the stent such that the assembly has both an inner and an outer membrane. The assembly is then subjected to a solvent such as dimethyl acetamide which will bond the coating and tube at their points of contact. The assembly is then cured in a forced air oven to remove any remaining solvent. The resulting bond is characterized in Figures 4A, 4B and 5D, showing a cross-section of a support member (11) encapsulated in coating, the coating (20) and graft member or membrane (21) seamlessly joined through the bonding process.

In another embodiment, the stent is first dip-coated in a 15% solids solution of silicone in xylene. The newly coated stent is cured to remove solvent. The coated stent is placed over or within an expanded porous membrane tube or both, and then either rolled down, expanded, or sequentially rolled down and then expanded to come into contact with the tube. The assembly is then exposed to a solvent, such as xylene, which will decrease the viscosity of the silicone such that it will migrate into the pores of the membrane. The stent-graft is then cured to drive off solvent and to secure the bond between the silicone and the porous membrane or membranes.

In another preferred method, the stent, the cross-section of which is depicted in Figure 7A, is spray coated with a polyurethane in solution. Following the coating process, the coated stent is cured to eliminate solvent. A cross-section of the coated stent is illustrated in Figure 7B. The coated stent is then loaded onto an expansion mandrel and, within an expanded PTFE tube, is expanded to its full diameter. The stent should be well-opposed to the tube wall. The coated stent and graft are carefully removed from the mandrel. Figure 7C depicts the relation of the stent support member (11), coating (20) and membrane (25) at this stage in the process.

A solvent which causes the coating to migrate into the pores of the tube is then introduced to bond the coated stent and graft. The entire device is then cured in a forced air oven to drive out any remaining solvent. The resulting bond is characterized in Figures 6B and 7D, which illustrate a cross-section of a coated support member and the region of bonding with the membrane. Extensions of the coating (26) are in interlocking engagement with the membrane as a result of the bonding method. Following the removal of solvent, the device is configured to its insertion diameter. The method could have either the alternative step or the added step of placing a tube within the stent prior to applying the solvent, such that the resulting assembly has a membrane on the interior of the stent or on both the interior and the exterior of the stent.

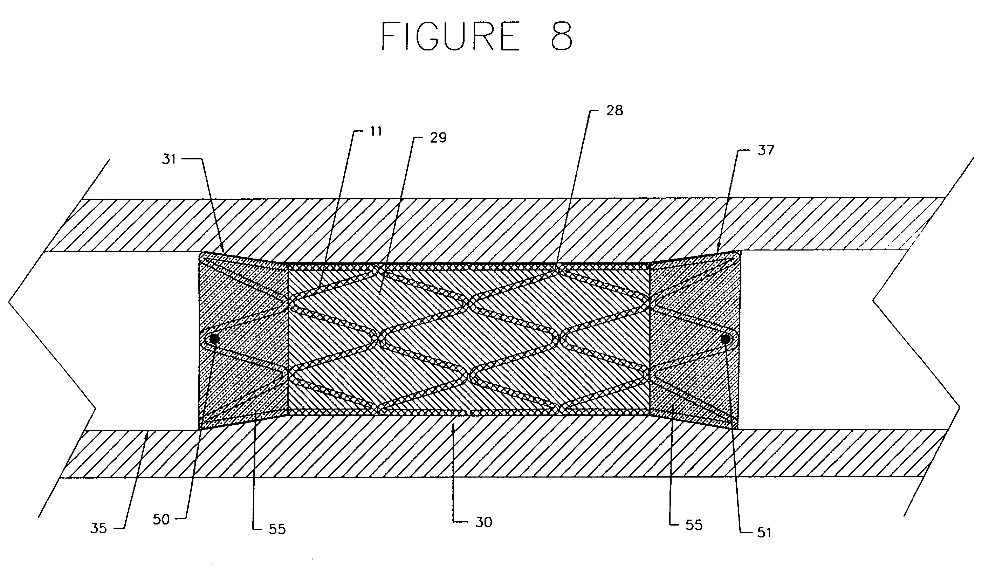

Referring to Figure 8, another embodiment of the invention is shown wherein the coating (28) is comprised of an elastomeric material and encapsulates substantially all the support members (11). The first membrane (29) covers only the medial region (30) of the stent-graft. The proximal and distal regions (31 and 37) comprise a second membrane (55), which is made of an elastomeric material. This second membrane substantially encapsulates the assembly at the proximal and distal regions. Trapped within the layers of the second membrane (55) is one or more radiopaque markers (50, 51). The proximal and distal regions (31 and 37) are able, during deployment and subject to the relative diameter of the vessel (35), to expand to a diameter greater than the diameter of the medial portion such that the device will effectively seat and seal within the vessel. The second membrane (55) may be formed by dipping the proximal and distal regions in an elastomeric material in solution in order to form an elastomeric membrane on the end elements which will facilitate sealing and positive apposition to the vessel wall. This embodiment is otherwise prepared in a manner which is consistent with any of the previous embodiments.

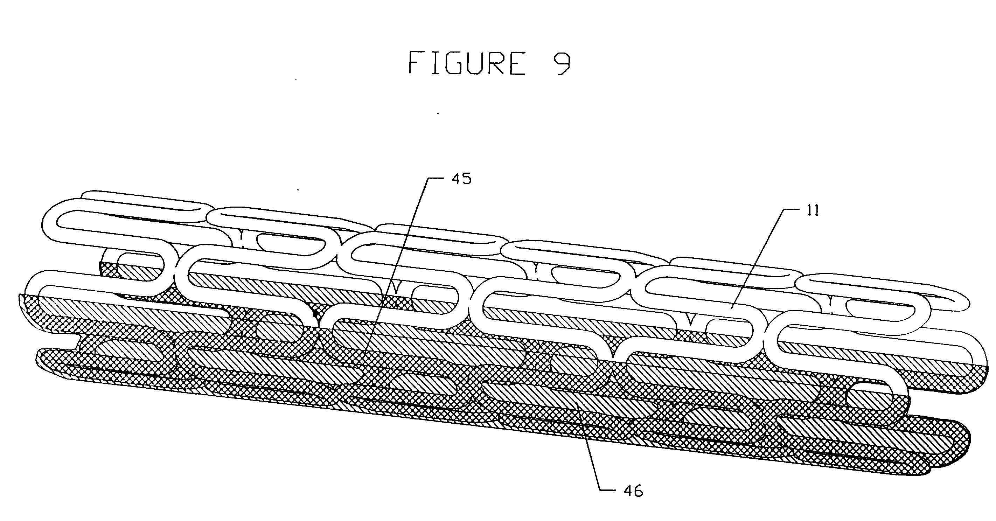

Turning now to Figure 9, an alternative embodiment of the invention is designed to occlude a side branch or aneurysm of the body lumen, and, if necessary, also provide support to the body lumen, while permitting flow through the main branch and/or to other side branches. Utilizing similar materials and techniques as those of the previous embodiments, but applying them to only approximately one-half of the stent body, the stent support members (11) comprise a coating (45) and a membrane (46). Although pictured in Figure 9 on the outside of the stent, the membrane may alternatively be on the inside of the stent, or on both the inside and the outside of the stent. The assembly may also comprise one or more radiopaque markers (not pictured). During deployment, the device is positioned such that the assembly provides radial support to a body lumen proximate to a side branch or aneurysm of that lumen. The portion of the assembly comprising the coating and membrane occlude the side branch and/or aneurysm while allowing blood flow on the opposite side. The coating and the membrane are bonded to one another in the same manner as in the previous alternative embodiments.

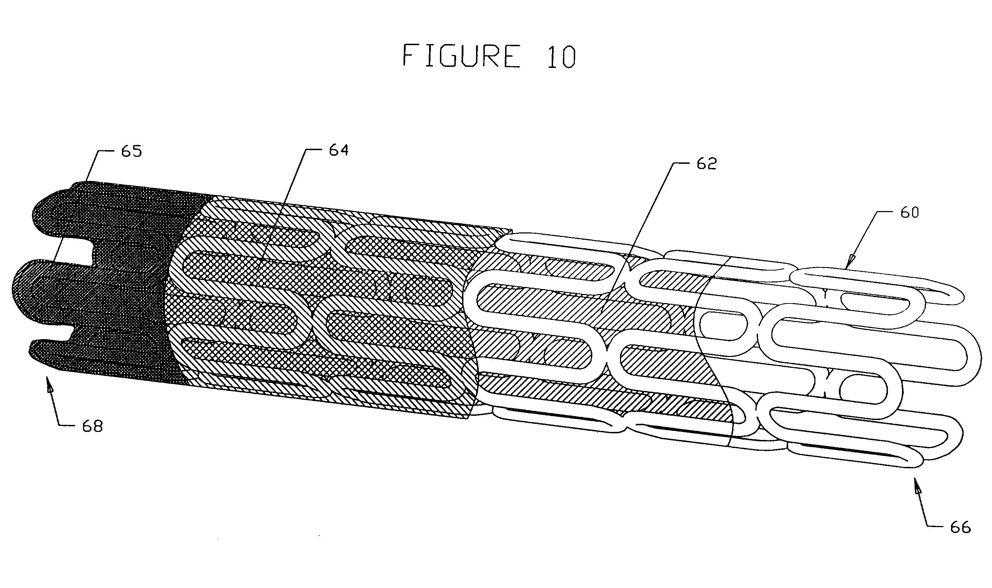

Figure 10 represents a partial progressive cut-away of another embodiment of the invention. In the embodiment illustrated in Figure 10, the assembly comprises a stent (60), a first membrane (62) defining a luminal surface, a second membrane (64) defining a vascular surface and a coating (65). Because Figure 10 is a progressive cut-away illustration, the coating is illustrated as substantially encapsulating the distal region (68) of the assembly, but in actuality encapsulates both the proximal and the distal regions. The membranes may be sintered or otherwise bonded to one another. The coating is bonded to the membranes in the same manner as in the previous embodiments.

### Example 1:

For the purpose of further illustration, an exemplary method for preparing a stent-graft assembly is described as follows. A GFX stent, which is manufactured by Arterial Vascular Engineering, Inc., in Santa Rosa, California, was first provided in a 18mm length. The end of the stent was mounted on a 0.109 inch mandrel. The stent was pre-heated at 80 degrees Celsius. The stent was then sprayed at a rate of 5 microliters per second for 5 seconds with a 5.0% solids solution of polyurethane in dimethyl acetamide

Polyurethanes which may be used in accordance with the present invention include segmented polycarbonate polyurethane such as that sold under the trademark CHRONOFLEX type AR, which is available from Cardiotech, Inc., located in Woburn, Massachusetts The coated stent was then cured for five minutes at 80 degrees C. The foregoing process was repeated an additional five times, with the respective end of the stent mounted on the mandrel alternated each time, thereby alternating each time the end of the stent exposed to the spray.

The stent was then expanded to a 4.5mm diameter over an expansion mandrel inside a 4mm ePTFE tube, available from Baxter Healthcare, Inc., located in Laguna Hills, California, such that the stent was well opposed to the graft wall. The stent and graft combination were then placed in a super-saturated atmosphere of dimethyl acetamide within an enclosed chamber. The device was then cured in a forced air oven at 80 degrees C for 1 hour. Following curing, the ends of the stent-graft were trimmed to remove graft material from between the peaks of the support members. The stent was then configured to its insertion diameter. Unitizing a fine-tipped syringe dispenser, a small drop of 5% polyurethane solution was placed on each stent peak to fully encapsulate the stent member at the peak. The assembly is again cured for one hour at 80 degrees C.

### Example 2.

To form a polyurethane tube, a mandrel was slowly dipped into a polymeric coating matrix. One specific coating matrix which is believed to be highly suitable for this coating procedure is a segmented polycarbonate polyurethane elastomer which is further provided in a dimethyl acetamide solvent. The mandrel was then slowly removed from the matrix. The coated mandrel was hung vertically and cured at 80 degrees C for 1 hour. Following the curing process, the newly formed polyurethane tube was carefully removed from the mandrel.

A MICRO STENT II stent, also manufactured by Arterial Vascular Engineering, Inc., was spray coated with five microliters per second for five seconds with the same polyurethane. The process was performed with the end of the stent mounted on a mandrel, repeated an additional five times, each time alternating the end of the stent which is in contact with the mandrel. Between each coat, the stent was cured for five minutes in a forced air oven at 80 degrees C. The coated stent was then cured in a forced air oven at 80 degrees Celsius for one hour, allowed to cool and then expanded within a polyurethane tube on an expansion mandrel to 4.5mm. The coated stent with graft were then exposed to dimethyl acetamide solvent via an atomizing spray for one second at a rate 100 microliters per second at 10 second intervals within an enclosed chamber at ambient temperature for 30 minutes.

The assembly was then cured at 80 degrees Celsius in a forced air oven for 1 hour. Following curing, the ends of the stent-graft were trimmed to remove graft material from between the peaks of the support members. The stent was then configured to its insertion diameter. The ends of the assembly were dipped up to the first stent member in 5% polyurethane solution, removed, and the assembly was then cured for one hour at 80 degrees C.

### Example 3

A GFX stent, available from Arterial Vascular Engineering, Inc., was expanded to its intermediate diameter utilizing an expansion mandrel and dip-coated with silicone in a 15% solids solution in xylene, the silicone available from Specialty Silicone located in Cupertino, California. The coated stent was then cured in a forced air oven at 80 degrees Celsius for one hour. The coated stent was then expanded within an ePTFE tube and was sprayed with xylene. The assembly was then cured in a forced air oven at 80 degrees Celsius for one hour. Following curing, the ends of the stent-graft were trimmed to remove graft material from between the peaks of the support members, and the stent was configured to its insertion diameter.

### Example 4:

A thin tape of ePTFE, approximately 0.0004 inch in thickness, was wound around a 3.25mm mandrel under slight tension at approximately a 60 degree angle to the longitudinal axis of the mandrel. Adjacent edges of the tape overlapped approximately 67 per cent of the tape's width. The wrapped mandrel was sintered at 370 degrees Celsius for 45 minutes.

A GFX stent, which is manufactured by Arterial Vascular Engineering, Inc., in Santa Rosa, California, was provided in a 18mm length. The end of the stent was mounted on a 0.109 inch diameter mandrel. The stent was pre-heated at 80 degrees Celsius. The stent was then sprayed at a rate of 6.3 microliters per second for 10 seconds with a 5.0% solids solution of polyurethane in dimethyl acetamide. The coated stent was then cured for 90 seconds at 100 degrees Celsius. The procedure was repeated with the opposite end of the stent mounted on the mandrel.

Polyurethanes which may be used in accordance with the present invention include segmented polycarbonate polyurethane such as that sold under the trademark CHRONOFLEX type AR, which is available from Cardiotech, Inc., located in Woburn, Massachusetts.

The thin ePTFE tube was removed from the mandrel and placed over the coated stent. The stent was then expanded to a 3.5mm diameter over an expansion mandrel while inside the thin tube previously prepared, such that the stent was well opposed to the graft wall. The stent and graft combination were then placed in a super-saturated atmosphere of dimethyl acetamide within an enclosed chamber. The device was then cured in a forced air oven at 80 degrees C for fifteen minutes. Following curing, the ends of the stent-graft were trimmed to remove graft material from between the peaks of the support members. The stent was then configured to its insertion diameter. Utilizing a fine-tipped syringe dispenser, a small drop of 5% polyurethane solution was placed on each stent peak to fully encapsulate the stent member at the peak. The assembly was again cured for thirty minutes at 80 degrees Celsius.

### Example 5:

Utilizing a thin, expanded polytetrafluoroethylene tape of approximately 0.0004 inch in thickness, the tape was wound helically around a mandrel from one end of the mandrel and progressing to the other end under slight tension at an angle of approximately 50 degrees to the longitudinal axis of the mandrel. The tape was wound a second time in the opposite direction to form a second layer, again at an angle of approximately 50 degrees to the longitudinal axis of the mandrel. Throughout each step of wrapping the tape, adjacent edges of the tape overlapped approximately 30 per cent. The wrapped mandrel was then sintered at a temperature of 370 degrees Celsius for forty-five minutes.

A GFX coronary bypass stent, which is manufactured by Arterial Vascular Engineering, Inc., was spray coated with five microliters per second for five seconds with segmented polycarbonate polyurethane. The process was performed with the end of the stent mounted on a mandrel, repeated an additional five times, each time alternating the end of the stent which was exposed to the spray. Between each coat, the stent was cured for five minutes in a forced air oven at 80 degrees C. The coated stent was then cured in a forced air oven at 80 degrees Celsius for one hour, and allowed to cool.

The thin ePTFE tube was removed from the mandrel and placed over the coated stent. The coated stent was then expanded within the prepared thin ePTFE tube on an expansion mandrel to 4.5mm. The coated stent with graft were then exposed to dimethyl acetamide solvent via an atomizing spray for one second at a rate 100 microliters per second at 10 second intervals within an enclosed chamber at ambient temperature for 30 minutes.

The assembly was then cured at 80 degrees Celsius in a forced air oven for 30 minutes. Following curing, the ends of the stent-graft were trimmed to remove graft material from between the peaks of the support members. The stent was then configured to its insertion diameter.

A stent-graft assembly and method of manufacturing a stent-graft assembly has been disclosed. Although the present invention has been described in accordance with the embodiments shown, one of ordinary skill in the art will readily recognize that there could be variations to the embodiments and those variations would be within the spirit and scope of the present invention.

A wide variety of suitable materials used for stents and grafts may be interchanged without diverging from the methods or structures of the invention claimed. For example, the type of stent utilized could be varied greatly. The embodiments disclosed herein focus on a stent comprising independent support members, but a stent which is comprised of a slotted tube or of a rolled film configuration may also be used. Further, suitable stents include stents made of nitinol or other shape memory alloy. In order to confer radiopacity on an alternative stent, various methods may be utilized. For example, a radiopaque metal marker such as Gold, Tantalum, Platinum, Iridium or any alloy thereof may be embedded or encapsulated into the coating of the device.

A further example of yet another manner of fabricating the stent-graft assembly involves wrapping a first tape about the mandrel. Loading the stent onto the mandrel over the first tape. Wrapping a second tape over the stent. And then applying a hot shoe proximate the interstices between the stent support members to sinter the two layers of tape together.

Suitable membrane material also may include autographs, which are vessels transplanted within the patient or host; allografts, which refer to vessels transplanted from a donor which is a member of the same species as the patient or host; or xenografts, which are transplanted from a donor which is not a member of the same species as the patient or host.

Further, the instant invention can also be used for indications other than repairing and/or providing radial support to a body lumen. Other examples include aneurysm isolation and vessel occlusion. The foregoing embodiments and examples are illustrative and are in no way intended to limit the scope of the claims set forth herein.

## Claims

1. A stent-graft assembly comprising:
a generally cylindrical stent comprising at least one support member, an exterior and an interior, a medial region, a proximal region and a distal region, said at least one support member defining a passageway through the stent;
a first coating substantially encapsulating at a least a portion of the at least one support member;
a first membrane, said membrane covering at least a portion of at least one of said exterior and interior; and
a plurality of bonding regions between the coating and the membrane.

2. The stent-graft assembly of claim 1 wherein said coating and said membrane define a unitary structure.

3. The stent-graft assembly of claim 1 wherein said membrane comprises pores and wherein said coating extends into said pores.

4. The stent-graft assembly of claim 1 wherein the membrane covers substantially all of at least one of the exterior or interior and the proximal and distal regions further comprise a second coating substantially encapsulating the assembly at the proximal and distal regions.

5. The stent-graft assembly of claim 1, the interior comprising a second coating at the proximal and distal regions, the second coating substantially encapsulating at least a portion of the at least one support member and at least a portion of the membrane at the proximal and distal regions.

6. The stent-graft assembly of claim 1 wherein the first coating is elastomeric, the first membrane covers only the medial region of the stent, and the assembly also comprises a second elastomeric membrane at the proximal and distal regions.

7. The stent-graft assembly of claim 6, wherein the second membrane substantially encapsulates the assembly at the proximal and distal regions.

8. The stent-graft assembly of claim 6, said stent comprising an expanded configuration wherein said medial region comprises a first diameter; and
said proximal and distal regions comprise a diameter which is greater than the diameter of the medial region.

9. A method of forming a stent-graft assembly, the method comprising the steps of:
providing a generally cylindrical stent having an interior and an exterior, first and second ends and at least one support member;
coating at least a portion of the at least one support member with a coating to substantially encapsulate at least a portion of the at least one support member;
providing at least one membrane;
placing said at least one membrane contiguous with at least a portion of at least one of the exterior and interior of the stent;
introducing a solvent in order to bond the membrane to the coating; and
removing the solvent.

10. The method of claim 9 wherein the step of coating the stent is performed by spraying a polymeric solution onto the stent.

11. The method of claim 9 wherein the step of coating the stent comprises a vapor deposition process.

12. The method of claim 9 wherein the step of coating the stent comprises dipping the stent into a polymeric solution.

13. The method of claim 9 wherein the step of coating the stent comprises: mounting an end of the stent on a rotating mandrel; heating the stent;
spraying the rotating stent with polymer in solution; curing the stent;
alternating the end of the stent that is mounted on the mandrel;
repeating the foregoing steps.

14. The method of claim 9 wherein the stent-graft assembly also comprises a medial region, a proximal region and a distal region, with the additional step of coating the proximal and distal regions of the stent-graft assembly.

15. The method of claim 9 wherein the coating and the membrane comprise chemically similar materials.

16. The method of claim 14 wherein the first membrane is placed to cover only the medial region of the exterior of the stent, and a second elastomeric membrane is placed over the proximal and distal regions of the assembly.

17. The method of claim 14 wherein the step of coating the distal and proximal regions comprises dipping the proximal and distal regions of the assembly into a polymer in solution.

18. The method according to claim 9 with the additional steps of preparing the at least one membrane, wherein said steps comprise:
preparing at least one tube by first providing a polymeric tape of less than 0.010 inch thickness;
winding the polymeric tape helically around at least a portion of the length of the mandrel in overlapping fashion; and
placing the wrapped mandrel in an oven at a sufficient temperature and for a sufficient time to achieve sintering of the overlapping portions of the tape.

19. The method according to claim 18 wherein the tape comprises a width and a plurality of edges, wherein the tape is wound around the mandrel such that adjacent edges of tape overlap 10-60 per cent of the width of the tape.

20. The method according to claim 18 wherein the tape is wound about the mandrel at an angle to the longitudinal axis of the mandrel, wherein the angle is between 30 and 60 degrees.

21. The method according to claim 18 wherein the wrapped mandrel is heated in an oven for between 30 and 45 minutes.

22. The method according to claim 9 wherein the step of introducing a solvent comprises placing the assembly within a super-saturated atmosphere of solvent.

23. A stent-graft assembly comprising:
a generally cylindrical stent having a proximal region and a distal region and comprising at least one support member, and at least one interstice, an exterior and an interior, said at least one support member defining a passageway through the stent;
a first membrane, said membrane contiguous with at least a portion of the interior of the stent to define a luminal surface;
a second membrane, said membrane covering at least a portion of the exterior of the stent to define a vascular surface, said first and second membrane bound to one another through at least one interstice of the stent; and
the assembly further comprising a proximal region and a distal region, said proximal and distal regions comprising a first coating on at least a portion of said proximal region and a second coating on at least a portion of said distal region.

24. A stent-graft assembly according to claim 23 wherein the first and second coating substantially encapsulates the first and second membranes and the support member or members at the proximal and distal regions.

25. The stent-graft assembly according to claim 23 wherein the first and second membrane are sintered to one another.

26. A method for preparing a stent-graft assembly, the method comprising the steps of:
lining at least a portion of the interior of a stent with a membrane, said stent comprising at least one interstice, a proximal region and a distal region;
placing a membrane over at least a portion of the exterior of a stent;
sintering the membranes to one another;
dipping the proximal and distal regions into a polymeric solution.

27. A stent-graft assembly comprising:
a generally cylindrical stent comprising at least one support member, an exterior and an interior, a medial region, a proximal region and a distal region, said at least one support member defining a passageway through the stent;
at least one membrane, said at least one membrane affixed to at least a portion of at least one of said interior and exterior, wherein the at least one membrane defines at least one of a luminal surface and a vascular surface, said at least one membrane being less than 0.040 inch thick.

28. The stent-graft assembly according to claim 27 wherein the membrane is less than 0.0016 inch thick.

29. The stent-graft assembly according to claim 27 wherein the at least one membrane is of generally uniform thickness.

30. The stent-graft assembly according to claim 27 wherein the stent further comprises:
a first polymeric coating substantially encapsulating at least a portion of the at least one support member; and
the at least one membrane is affixed to the coating.

31. The stent-graft assembly according to claim 30 wherein the at least one membrane is porous and the coating extends into the pores of the membrane.

32. The stent-graft assembly according to claim 30 wherein the at least one membrane and the coating form a unitary structure.

33. The stent-graft assembly according to claim 27 wherein the stent comprises at least one interstice, and the assembly further comprises a first inner membrane defining a luminal surface and a second outer membrane defining a vascular surface, wherein the first and second membranes are bound together through the at least one interstice.

34. The stent-graft assembly according to claim 33 wherein the first membrane is sintered to the second membrane.

35. The stent-graft assembly of claims 1, 23 or 27 wherein the membrane is a polymer selected from the group consisting of polyurethane, ePTFE, dimethyl terephthalate, polyester, polyethylene terephthalate and silicone.

36. The stent-graft assembly of claims 1, 23 or 27 wherein the coating is a polymer selected from the group consisting of polyurethane, flourinated ethylene propylene and silicone.

37. The stent-graft assembly of claims 1, 23 or 27 wherein the membrane or the coating also comprises at least one therapeutic agent.

38. The stent-graft assembly of claims 1, 23 or 27 wherein the assembly also comprises at least one radiopaque marker.

39. The stent-graft assembly of claims 1, 23 or 27 wherein the membrane comprises a primary diameter and wherein the membrane is distensible over a range of between 7 and 100% beyond the primary diameter.

40. A method for preparing a stent-graft assembly, the method comprising the steps of:
providing a polymeric tape of less than 0.010 inch thickness;
helically winding the tape about a mandrel in overlapping fashion;
heating the wrapped mandrel at sufficient temperature for sufficient time to achieve sintering of the overlapping portions of the tape to prepare a first thin-walled membrane;
removing the tube from the mandrel;
repeating the foregoing steps to prepare a second thin-walled membrane;
providing a generally cylindrical stent having an interior and an exterior, a proximal region and a distal region, at least one interstice and at least one support member;
lining at least a portion of the interior of the stent with the first membrane to define a luminal surface;
placing the second membrane over at least a portion of the exterior of the stent to define a vascular surface; and
bonding the first and second membranes to one another through the at least one interstice of the stent.

41. The method according to claim 40 with the added step of dipping the proximal and distal regions of the assembly into a polymeric solution.

42. The method according to claim 40 wherein the step of bonding the first membrane to the second membrane comprises heating the assembly for sufficient time at sufficient temperature to achieve sintering of the membranes to one another through the at least one interstice.

43. A method for preparing a thin-walled graft tube, the method comprising the steps of:
providing a polymeric tape of less than 0.010 inch thickness;
helically winding the tape about a mandrel in overlapping fashion;
applying pressure to the wrapped mandrel; and
heating the wrapped mandrel under pressure at a sufficient temperature for a sufficient time to achieve sintering of the overlapping portions of the tape.

44. The method of claims 9, 15 or 30 wherein the coating is a polymer selected from the group consisting of polyurethane, silicone and flourinated ethylene propylene.

45. The method of claims 9, 16, 18 or 26 wherein the membrane is a polymer selected from the group consisting of ePTFE, polyurethane, dimethyl terephthalate, polyester, polyethylene terephthalate, polyethylene terephthalate and silicone.

46. The method of claims 9, 31 or 40 wherein the coating or the membrane comprises at least one therapeutic agent.

47. The method of claims 9, 27 or 40 with the additional step of adding a radiopaque marker to the coating or the membrane.
